# EUROPEAN PATENT APPLICATION

(11) **EP 0 941 739 A1**
(43) Date of publication of application: **15.09.1999**
(21) Application number: 98830131.3
(22) Date of filing: 10.03.1998
(51) Int. Cl.: A61L 27/00

(54) **Porous coated artificial implant material and corresponding preparation process**

(71) Applicant: Bravo, Carlos, Armenia (CO); Mannino, Enzo, 40193 Palermo (IT)
(72) Inventor: Bravo, Carlos, Armenia (CO); Mannino, Enzo, 40193 Palermo (IT)
(74) Representative: De Nova, Roberto

(57) **Abstract**

This invention relates to an artificial implant material and a corresponding preparation process. The implant material comprises a porous polymer substrate coated with a polymer composition of polyurethane and polylactic acid and the preparation process comprises the stages of: immersing the porous polymer substrate in a solution containing polyurethane and polylactic acid, removing the porous substrate from the said solution and immersing the said substrate in a solvent in which the said polyurethane and the said polylactic acid are insoluble, and bringing about precipitation of the two polymers onto the surface of the said substrate. The implant material according to the invention can advantageously be used for the preparation of small diameter prostheses, in particular vascular prostheses for use in microsurgery on malfunctioning blood vessels.

## Description

This invention relates to an artificial material suitable for implanting into a living organism and a corresponding preparation process.

This invention also relates to an artificial prosthesis comprising the said material, in particular a small diameter vascular prosthesis which is suitable for use in microsurgery on malfunctioning blood vessels.

It is known that in the field of vascular microsurgery situations frequently arise where it is necessary to insert prostheses in order to correct defects in veins or arteries, such as e.g. in cerebral and coronary revascularisation, in the post-trauma reimplantation of limbs, in the elongation of vascular stems in reconstructive surgery and in transplants.

The conventional way of satisfying the above requirements is to use venous inserts taken from suitable veins in the patient, mainly from the saphenous vein.

However, the removal of lengths of vein is only possible in some patients who are free from complications such as phlebitis, post-radioactive conditions, vascular obliterating disease, intravenous chemotherapy, trauma, iatrogenic damage or other degenerative processes, and venous inserts often undergo a series of structural changes which can cause hyperplasia of the intima and arteriosclerosis.

In addition to this, the removal of a length of vein causes an additional wound for the patient and makes it necessary to prolong the operation when only one operating group is performing it.

For these reasons a variety of different artificial vascular prostheses have been developed which can be used as an alternative to venous inserts for modifying or replacing malfunctioning natural blood vessels.

These comprise tubular fabrics of a specific internal diameter which are formed of braided fibres of biocompatible synthetic polymers such as e.g. some polyesters and polyurethanes and polytetrafluoroethylene.

However, only large diameter artificial prostheses have found some use in vascular surgery, while in work on coronary arteries or peripheral blood vessels, where vascular prostheses of a diameter less than 6 mm often have to be used, satisfactory results which would make artificial prostheses preferable to venous inserts have not been obtained.

The main reason for the failure of vascular prosthesis implants of the abovementioned type is the high thromoogenicity of the materials used in their manufacture.

It has in fact been found that, after implanting, groups of platelets tend to adhere to the inside surface of the prosthesis in contact with the blood flow, and as they accumulate they initiate a thrombotic process which proliferates within the blood vessel until such time as it is occluded.

Furthermore, as a result of insertion of the aforesaid prostheses neointimal hyperplasia of the vessel can occur, that is progressive inward growth of natural tissues, principally smooth muscle cells which over time also tend to occlude the blood vessel.

A factor which is of considerable importance in the production of a vascular prosthesis is also the porosity of the material used.

In fact, in order that artificial vascular prostheses should effectively perform the function of allowing blood to circulate for a long period, the inside surface of the prostheses must undergo a rapid process of endothelialization after insertion, that is growth of layers of endothelial cells adhering to this inside surface which prevent the formation of thrombi and maintain a high degree of patency in the blood vessel.

In this connection various research workers have found that endothelial cells do not originate as a primary structure on the inside surface of an artificial prosthesis but are formed subsequent to the formation of aggregates adhering to the inside surface of the prostheses comprising fibroblasts and other cells which have migrated from the exterior through the porous wall of the prosthesis and/or have grown within the pores of the said wall.

However, in the case of small diameter vessels, the presence of platelets adhering to the inside surface of the prosthesis tends to occlude the pores of the constituent material, thus discouraging the process of endothelialization.

In conclusion, because of the abovementioned disadvantages the aforesaid small diameter vascular prostheses show a very much reduced level of patency, generally less than 50%, a short time after transplant, which makes them inapplicable in vascular microsurgery.

In order to overcome the abovementioned disadvantages considerable efforts have been made to confer antithrombogenic properties on the materials used in the production of vascular microprostheses.

In this respect artificial vascular prostheses comprising a polymer onto whose internal surface an antithrombogenic agent is chemically or reversibly bound have been developed.

However, endothelial cells do not grow in such prostheses, and the antithrombogenic agent only temporarily prevents the formation of thrombi, which form in any event and occlude the blood vessel after a given length of time following implant.

Alternatively, artificial vascular prostheses in which the internal surface is coated with materials which encourage adhesion by endothelial cells, such as gelatin or collagen, to which an antithrombogenic substance (e.g. heparin) is bound, have also been developed to prevent the formation of thrombi before the growth of endothelial cells.

However, in such prostheses neither the suppression of thrombi nor the formation of endothelial cells has proved sufficient, with the consequence that they do not have satisfactory patency after implant.

The technical problem which is fundamental to this invention is that of providing an artificial material for implants which is suitable for use in the production of small diameter prostheses, in particular small diameter vascular prostheses for microsurgery on malfunctioning blood vessels, without incurring the previously mentioned disadvantages in connection with the vascular prostheses of the known art, that is an artificial material for implants which has excellent antithrombogenic properties and a high capacity to encourage the growth of endothelial cells on its surface so that it retains a high degree of patency over a long period when used as small diameter prostheses.

This problem is resolved by an artificial implant material comprising a porous polymer substrate coated with a polylactic acid and polyurethane polymer composition.

In the artificial material according to the invention the porous substrate may be a fabric in the form of a hollow cylinder obtained by braiding filaments of a biocompatible and non-biodegradable synthetic polymer on one side.

The abovementioned braiding is preferably performed with multifilaments of synthetic polymer having a weight of 50 to 250 denier using a needle braiding machine.

The synthetic polymer comprising the substrate should have a high resistance to the pressure of the blood flow and be stable for long periods of time after being implanted in the living organism.

It may be advantageously selected from the group comprising polyesters of terephthalic acid such as polyethylene terephthalate, polypropylene terephthalate, polybutylene terephthalate and their derivatives.

Polyethylene terephthalate is particularly preferred as a polymer for construction of the porous substrate.

In accordance with a preferred embodiment of the invention the artificial material comprises a tubular fabric obtained by braiding on a single side polyethylene terephthalate multifilaments coated with a polylactic acid and polyurethane polymer composition.

Polyurethane is preferably present in the polymer composition in a percentage by weight of between 50 and 90% and as a segmented polyurethane.

In applications of the material according to the invention as a small diameter vascular prosthesis the size of the pores in the said material is an important aspect.

In fact, it is known that an artificial vascular prosthesis must have a porosity such as to allow fibroblasts and other cells which promote the growth of endothelial cells to migrate from the outside to the surface, at the same time avoiding excessive permeability to blood, which would favour the formation of thrombotic complexes.

In the artificial material according to the invention the optimum size of the pores is obtained by coating the porous substrate with one or more layers of the polymer composition.

Thus, in the applications of the material according to the invention as small diameter vascular prostheses, the pore size varies from 30 to 50 microns.

A pore size of 37 to 43 microns is particularly preferred for the artificial material according to the invention.

The layers of the polymer composition also define the final thickness of the walls of the artificial material according to the invention, which vary from 50 microns to 250 microns, preferably from 100 microns to 150 microns.

The artificial material according to the invention may be prepared by means of a process which comprises first immersing the porous substrate in a solution containing polyurethane and polylactic acid and secondly immersing the moistened substrate in a solvent in which the two polymers are not very soluble, thus bringing about precipitation of the two polymers onto the porous substrate.

preferably, the solvent used for precipitation of the two polymers is ethanol.

The solution of polylactic acid and polyurethane can be prepared by dissolving appropriate quantities of the two polymers in a suitable solvent or preferably by mixing predetermined volumes of separate solutions of the two polymers in suitable solvents.

In the latter case polyurethane is preferably dissolved in a mixture of N-dimethyl formamide and tetrahydrofuran in the ratio of 3:1 and the polylactic acid is dissolved in chloroform.

Particularly preferred is a solution of polyurethane and polylactic acid obtained by mixing predetermined volumes of a saturated solution of polyurethane in N-dimethyl formamide and tetrahydrofuran in the ratio 3:1 and a saturated solution of polylactic acid in chloroform.

The fabric may be immersed in the polymer coating solution and the precipitation solvent several times so as to obtain an artificial material with an adequate pore size and wall thickness.

It has been found that the artificial material according to the invention has excellent antithrombogenic properties and a greater ability to encourage the growth of endothelial cells on its surface in a short time.

This has been advantageously used in the preparation of small diameter artificial prostheses, in particular vascular prostheses which have given optimum results when implanted in living organisms.

In fact it has been found that vascular prostheses prepared using the material according to the invention, once implanted in a living organism, do not tend to cause thrombotic complexes to develop and advantageously promote the growth of endothelial cells on their surfaces and restructuring of the intima of the blood vessel to which they are applied, within a short time.

Thus, vascular prostheses prepared using the material according to the invention retain a high degree of patency after implantation, not less than 70% of which is also maintained for long periods after making the implant.

The features and advantages of this invention will be even more apparent from the following description of an example of a preparation of artificial material according to the invention and embodiments of vascular prostheses prepared using the material according to the invention, examples which are given purely for indication and are not restrictive.

In the figures:
Figures 1, 2 are photographic reproductions of part of the internal surface of a vascular prosthesis prepared using the material according to the invention magnified by 52 and 200 times respectively,
Figures 3, 4, 5 are photographic reproductions of a length of the outside surface of the prosthesis in Figures 1 and 2 magnified by 56, 580 and 1600 times respectively,
Figure 6 is a photographic reproduction of a length of the internal surface of the vascular prosthesis in the foregoing figures 50 days after implant, magnified 32 times.

A fabric was formed in the shape of a hollow cylinder using a needle braiding machine, by braiding multifilaments of polyethylene terephthalate weighing from 50 to 250 denier, on one side only.

This fabric was 10 mm long and had an internal diameter of 2 mm.

A saturated solution of segmented polyurethane (pelletane 2363) in a mixture of N-dimethyl formamide and tetrahydrofuran in the ratio 3:1 and a saturated solution of polylactic acid in chloroform were also prepared.

Then 80 parts by volume of the solution containing the segmented polyurethane were mixed with 20 parts by volume of the solution containing the polylactic acid.

Subsequently, the polyethylene terephthalate fabric was first immersed in the said polyurethane and polylactic acid solution, removed from the solution and again immersed in ethanol, where it was held for the length of time necessary to cause precipitation of a thin layer of the two polymers on the surface of the fabric.

In this way an artificial material was obtained which was then removed from the solvent, dried and sterilized in accordance with conventional procedures.

The aforesaid material had a pore size of 40 microns, a wall thickness of 100-150 microns and the surface layer which covered the polyethylene terephthalate fabric consisted of polyurethane and polylactic acid.

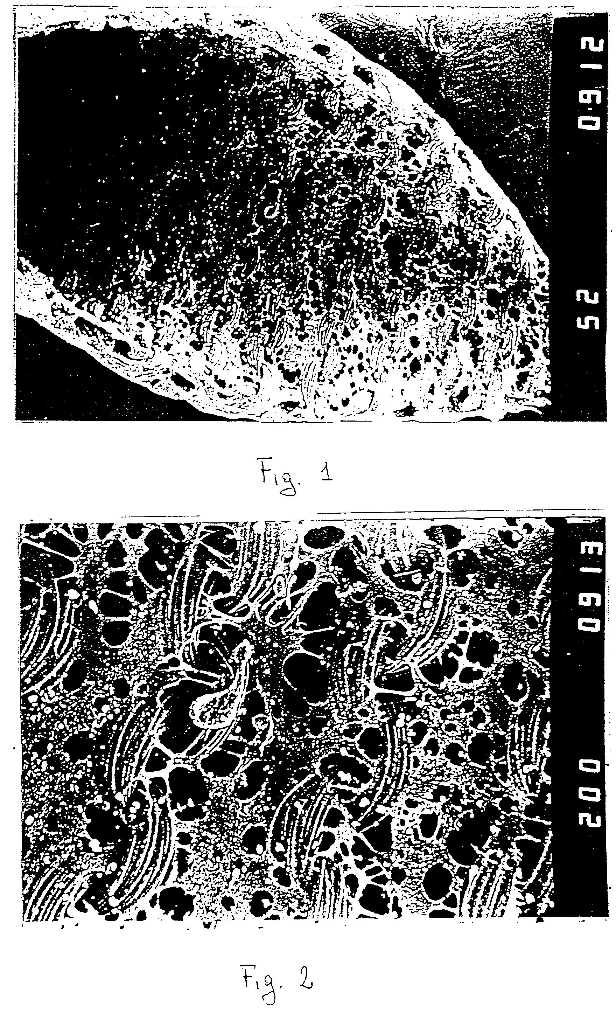

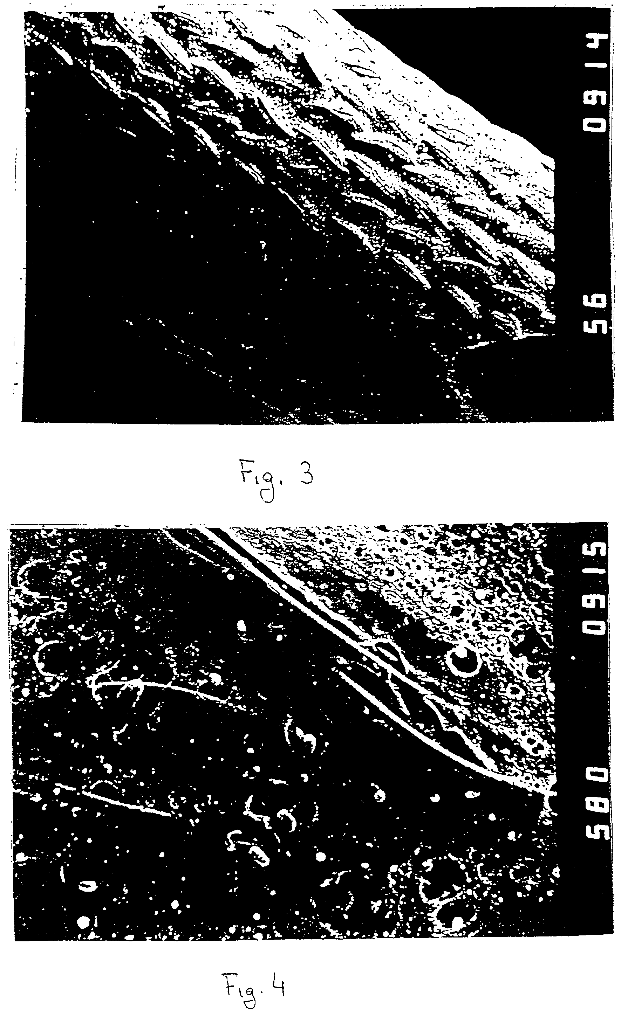

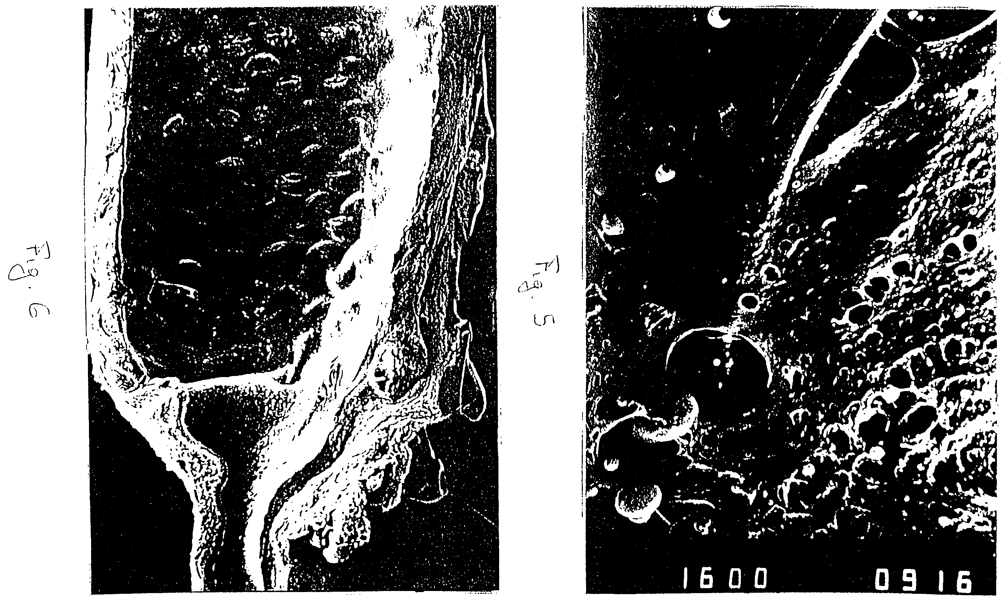

The thin layer of the polyurethane and polylactic acid composition on the outer and inner surfaces of the prosthesis are clearly visible in Figures 1-5.

The artificial material obtained in this way was used as a small diameter vascular prosthesis in laboratory animals.

### EXAMPLE 1

Twenty vascular prostheses obtained as above were implanted in the abdominal aorta of twenty male rats weighing 400 - 450 g. After implant the patency of the implanted prostheses was assessed one, two and six months after implant and the result was always around 100%.

Histological examinations of the prostheses were made 50, 68 and 180 and 365 days after implant, the removed prostheses washed with saline solution and fixed in phosphate-buffered 2% glutaraldehyde being subjected to examination under an optical microscope and a transmission and scanning electron microscope.

From the abovementioned examinations it was found that only 50 days after implant the internal surface of the prosthesis was completely covered with flat endothelial cells, which means that the intima of the artery was completely reconstructed, as may be seen in Figure 6.

In addition to this the internal surface of the prosthesis appeared smooth, without any signs of thrombosis and the lumen was completely open. The outer surface on the other hand was covered with a fibrous layer with numerous fibroblasts and collagen fibrils.

68 days following implant the structure of the prosthesis remained substantially unchanged except that the layer of endothelial cells on the internal surface was very much more obvious.

180 days following implant the internal surface of the prosthesis remained smooth and groups of cells having an elongated nucleus similar to smooth muscle cells were obvious. The external surface was covered with a thick organized fibrous layer which was thicker at the cranial anastomosis.

After 365 days following implant the results of the histological examinations were entirely similar to those found 180 days after implant.

### EXAMPLE 2 (comparison example)

Ten vascular prostheses made of the polyethylene terephthalate fabric used for preparation of the prostheses in Example 1 coated with one layer of polyurethane were prepared.

The abovementioned fabric was 10 mm long and had an internal diameter of 2 mm.

Coating was performed by immersing the polyethylene terephthalate fabric in a solution of polyurethane in 3:1 N-dimethyl formamide-tetrahydrofuran, removing the moistened fabric from that solution and immersing the said moistened fabric in ethanol for a sufficient time to cause precipitation of the polyurethane onto the surface.

The vascular prostheses obtained in this way had a pore size of 10-15 microns and a wall thickness of 100-120 microns.

The vascular prostheses prepared as above were implanted in the abdominal aorta of ten male rats weighing 400-450 g. 1 day following implant a patency of 80% was found and after 2 days following the operation only 50% of the prostheses remained open on account of the formation of thrombi.

The patency of the prostheses were also assessed after 1 and 2 months following implant, and was found to be 30%.

Histological examinations of the prostheses were made 62 days after implant, subjecting the prostheses removed, washed with saline solution and fixed in phosphate-buffered 2% glutaraldehyde to examination under an optical microscope and a transmission and scanning electron microscope.

From the abovementioned examinations it was found that there were continuous layers of fusiform cells and groups of leukocytes without any formation of endothelial cells on the internal surfaces of the prostheses.

### EXAMPLE 3 (comparison example)

Ten vascular prostheses made of the polyethylene terephthalate fabric used for preparation of the prostheses in Example 1 coated with 1 layer of polylactic acid were prepared.

The said fabric was 10 mm long and had an internal diameter of 2 mm.

The fabric was coated by first immersing the polyethylene terephthalate fabric in a solution of polylactic acid in chloroform, removing the moistened fabric from the said solution and immersing the said moistened fabric in ethanol for a sufficient time to cause precipitation of the polylactic acid onto its surface.

The vascular prostheses obtained in this way had a pore size of 60 microns and a wall thickness of 100-120 microns.

Ten vascular prostheses prepared as above were implanted in the abdominal aorta of ten male rats weighing 400-450 g. 1 day after implant the prostheses were found to have a patency of 80% and two days after the operation only 40% of the prostheses remained open on account of the formation of thrombi.

The patency of the prostheses was also assessed after 1 and 2 months following implant, and found to be 20%.

Histological examinations of the prostheses were made 2, 19 and 49 days after implant, the prostheses removed, washed with saline solution and fixed in phosphate-buffered 2% glutaraldehyde being subjected to examination under an optical microscope and a transmission and scanning electron microscope.

From the abovementioned examinations it was obvious after 2 days following implant that thrombi consisting of fibrin, erythrocytes, leukocytes and groups of platelets had formed within the lumen of the prostheses, and that 19 days following implant these occluded the abovementioned prostheses.

After 49 days following implant the lumen of the prostheses was covered with approximately 20 layers of flattened cells (fibroblasts), collagen and elastic fibres.

### EXAMPLE 4

With the process used to prepare the vascular prostheses in Example 1, ten vascular prostheses were prepared according to the invention with a pore size of 37 microns and a wall thickness of 100 - 120 microns.

The fabric of the aforesaid prostheses was that used for preparation of the prosthesis in Example 1, 10 mm long and had an internal diameter of 2 mm.

The prostheses prepared as above were implanted in the abdominal aorta of 10 male rats weighing 400-450 g. After implant the patency of the implanted prostheses was assessed after periods of one, two and six months following implant, and was always found to be around 100%.

Histological examinations of the prostheses were performed 50, 70, 180 and 365 days after implant with the prostheses removed, washed with saline solution and fixed in phosphate-buffered 2% glutaraldehyde being subjected to examination under an optical microscope and transmission and scanning electron microscope.

The results of the aforesaid examinations were wholly comparable to those of the corresponding histological examinations of the prosthesis in Example 1.

## Claims

1. Artificial implant material comprising a porous polymer substrate coated with a polymer composition of polyurethane and polylactic acid.

2. Artificial material according to Claim 1, in which the said porous substrate is a fabric obtained by braiding filaments of a biocompatible non-biodegradable polymer.

3. Artificial material according to Claim 2, in which the said braiding is performed with multifilaments of the said polymer weighing from 50 to 250 denier.

4. Artificial material according to either of Claims 2 and 3, in which the said polymer is a polyester of terephthalic acid selected from the group comprising polyethylene terephthalate, polypropylene terephthalate, polybutylene terephthalate and their derivatives.

5. Artificial material according to Claim 4, in which the said polyester of terephthalic acid is polyethylene terephthalate.

6. Artificial material according to Claim 5, characterised in that it is in the form of a hollow cylinder.

7. Artificial material according to Claim 6, in which the said polyurethane is a segmented polyurethane.

8. Artificial material according to Claim 6 or 7, in which the polyurethane of the polymer composition is present in percentages by weight of from 50% to 90%.

9. Artificial material according to any one of Claims 6 to 8, characterised in that the pore size lies in the range 30-50 microns.

10. Artificial material according to Claim 9, characterized in that the pore size lies in the range 37 - 43 microns.

11. Artificial material according to any one of Claims 6 to 10, characterized in that the wall thickness is from 50 microns to 250 microns.

12. Artificial material according to Claim 11, characterized in that it has a wall thickness of from 100 microns to 150 microns.

13. Implant prosthesis made of an artificial material according to any one of the foregoing claims.

14. Prosthesis according to Claim 13, characterized in that it is a vascular prosthesis of hollow cylindrical shape with an internal diameter of not more than 6 mm.

15. Process for preparation of the artificial material according to any one of Claims 1 to 12, characterised in that it comprises the stages of:
- immersing the porous polymer substrate in a solution containing polyurethane and polylactic acid,
- removing the porous substrate from the said solution and immersing the said substrate in a solvent in which the said polyurethane and said polylactic acid are insoluble, bringing about precipitation of the two polymers onto the surface of the said substrate,
- if necessary repeating the previous stages in order to obtain an artificial material having the desired pore size and wall thickness.

16. Process according to Claim 15, in which the said solution is prepared by mixing predetermined volumes of a solution of polyurethane in a mixture of N-dimethylformamide and tetrahydrofuran in the ratio 3:1 and a solution of polylactic acid in chloroform.

17. Process according to Claim 16, in which the said solutions of polylactic acid in chloroform and of polyurethane in N-dimethylformamide and tetrahydrofuran are saturated solutions.

18. Process according to Claim 17 in which the said solvent is ethanol.

19. Polymer composition comprising polyurethane and polylactic acid for use in coating a porous polymer substrate for the preparation of an artificial implant material.

20. Polymer composition according to Claim 19, in which the polyurethane is present in percentages by weight of from 50% to 90% and the polylactic acid is present in percentages by weight of from 10% to 50%.

21. Use of a mixture of polylactic acid and polyurethane for coating an artificial implant material.
